# EUROPEAN PATENT APPLICATION

(11) **EP 3 520 677 A1**
(43) Date of publication of application: **07.08.2019**
(21) Application number: 19153696.0
(22) Date of filing: 25.01.2019
(51) Int. Cl.: A61B 1/12, A61B 17/00

(54) **INTRAOPERATIVE LENS CLEANING DEVICE**

(30) Priority: 26.01.2018 US 201815880583
(71) Applicant: Covidien LP, Mansfield, MA 02048 (US)
(72) Inventor: AHMED, Mushtaque Syed, 500057 Hyderabad (IN); MUJAWAR, Arifmohamad Hamaju, 416301 Sangli (IN); MADDUR, Jeevan Shankar Setty, 560096 Bangalore (IN); VIKHARANKAR, Yogesh Kishore, 423108 Maharashtra (IN)
(74) Representative: Maschio, Antonio

(57) **Abstract**

An intraoperative lens cleaning device includes a handle component and a tool assembly supported on a distal portion of the handle component. The handle component includes a grip portion and an elongate body portion. The tool assembly includes a distal loop portion that defines an opening and is formed of a resilient material that is deformable to a deformed configuration to be received within a trocar-cannula assembly. A cleaning member is formed from a biocompatible material and is supported on the distal loop portion to cover the opening.

## Description

### BACKGROUND

### 1. Technical Description

The present disclosure is directed to a lens cleaning device and, more particularly, to a lens cleaning device that is configured to access a body cavity and to clean an endoscope lens of an endoscope during an endoscopic surgical procedure.

### 2. Background of Related Art

During an endoscopic surgical procedure, surgical tools are inserted through small incisions in a patient to access an operative site within a body cavity. Typically, the body cavity is insufflated with an inert gas such as CO₂ and an endoscope is inserted through one of the incisions to allow a clinician, e.g., a surgeon, to visualize the surgical site. Visualization of the surgical site is critical to successfully performing the surgical procedure.

During the surgical procedure, it is common for a lens of the endoscope to become smudged and/or obstructed with debris and require cleaning. Typically, the endoscope is removed from the body cavity to clean the endoscope lens. However, removal of the endoscope from the body cavity during the surgical procedure causes a temporary loss of visualization of the surgical site, increases the time required to perform the surgical procedure, and may result in loss of insufflation within the body cavity.

A continuing need exists in the surgical arts for a method and device to clean the lens of an endoscope within a body cavity during a surgical procedure.

### SUMMARY

One aspect of the disclosure is directed to an intraoperative lens cleaning device that includes a handle component and a tool assembly. The handle component includes a grip portion and an elongate body portion that together define an internal bore. The elongate body portion extends distally from the grip portion and includes a distal portion having a first coupling member. The tool assembly includes a proximal body portion that defines an internal bore and a distal loop portion. The internal bore of the proximal body portion communicates with the internal bore of the handle component. The proximal body portion includes a second coupling member that is configured to engage the first coupling member to releasably couple the tool assembly to the handle component. The distal loop portion defines an opening and is formed of a resilient material that is deformable to a deformed configuration to be received within a trocar cannula assembly. A cleaning member is formed from a biocompatible material and is supported on the distal loop to cover the opening.

In embodiments, the cleaning member is formed of an absorbent material.

In some embodiments, the cleaning member is formed of a biocompatible fabric.

In certain embodiments, the cleaning member is formed of cotton fibers.

In some embodiments, the cleaning member is formed of a transparent material.

In embodiments, the proximal body portion of the tool assembly has a diameter "D" and the distal loop portion has a width "W" that is greater than the diameter "D".

In some embodiments, "W" is 5 to 10 times greater than "D".

In certain embodiments, "W" is 4 to 8 times greater than "D".

In embodiments, the grip portion includes a slip-resistant covering.

In some embodiments, the slip-resistant covering is formed from rubber.

In certain embodiments, the device includes a fluid delivery member that is supported within the internal bore of the handle component and the internal bore of the proximal body portion of the tool assembly. The fluid delivery member includes a proximal portion and a distal portion that is positioned adjacent to the cleaning member.

In embodiments, the fluid delivery device includes a capillary tube.

In some embodiments, the fluid delivery device includes a wicking member.

In certain embodiments, the handle component includes a fluid inlet port that communicates with a proximal portion of the fluid delivery member.

In some embodiments, the first and second coupling members include first and second screw threads, respectively.

In certain embodiments, the device includes a fluid suction port and a fluid suction tube. The fluid suction port communicates with a proximal end of the fluid suction tube and the fluid suction tube has a distal end in fluid communication with the cleaning member of the tool assembly.

Another aspect of the present disclosure is directed to a method of performing a surgical procedure including inserting an endoscope through a first incision into a body cavity of a patient; inserting a cleaning device through a second incision into the body cavity of the patient to position a tool assembly of the cleaning device adjacent a lens of the endoscope; and manipulating the cleaning device to position a cleaning member of the tool assembly into contact with the lens of the endoscope within the body cavity to clean the lens of the endoscope within the body cavity.

In embodiments, the method includes supplying a cleaning solution to the cleaning member through a delivery member of the cleaning device.

In some embodiments, inserting the cleaning device through the second incision includes inserting the cleaning device through a 5mm trocar cannula assembly positioned within the second incision.

In embodiments, inserting the cleaning device through the 5mm trocar cannula assembly includes deforming the cleaning member of the cleaning device from a dimension greater than the diameter of the 5mm trocar cannula assembly to a dimension smaller than the diameter of the 5mm trocar cannula assembly.

### BRIEF DESCRIPTION OF THE DRAWINGS

Various embodiments of the presently disclosed intraoperative lens cleaning device are described herein below with reference to the drawings, wherein:
FIG. 1 is a side perspective view of an exemplary embodiment of the presently disclosed intraoperative lens cleaning device;
FIG. 2 is an exploded, side perspective of the intraoperative lens cleaning device shown in FIG. 1;
FIG. 3 is a cross-sectional view taken along section line 3-3 of FIG. 1;
FIG. 4 is an enlarged view of the indicated area of detail shown in FIG. 3;
FIG. 5 is a cross-sectional view taken along section line 5-5 of FIG. 1; and
FIG. 6 is a perspective view of the intraoperative lens cleaning device positioned through a trocar cannula assembly to a position within a body cavity adjacent a lens of an endoscope.

### DETAILED DESCRIPTION OF EMBODIMENTS

The presently disclosed intraoperative lens cleaning device will now be described in detail with reference to the drawings in which like reference numerals designate identical or corresponding elements in each of the several views. However, it is to be understood that the disclosed embodiments are merely exemplary of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

In this description, the term "proximal" is used generally to refer to that portion of the device that is closer to a clinician, while the term "distal" is used generally to refer to that portion of the device that is farther from the clinician. In addition, the term "endoscopic" is used generally used to refer to endoscopic, laparoscopic, arthroscopic, and/or any other procedure conducted through a small diameter incision or trocar cannula assembly. In addition, the term "clinician" is used generally to refer to medical personnel including surgeons, doctors, nurses, and support personnel.

The presently disclosed lens cleaning device is configured for intraoperative use and facilitates cleaning of a lens of an endoscope without having to remove the endoscope from a body cavity. The presently disclosed device includes a tool assembly that is removably coupled to a distal portion of a handle component. The distal portion of the handle component and the tool assembly are configured to be received through a small diameter trocar/cannula assembly, e.g. 5mm or 10mm trocar-cannula assembly. The tool assembly includes a proximal body portion and a distal loop that supports a cleaning member. The distal loop is formed of a deformable and resilient material to facilitate insertion of the tool assembly through the trocar-cannula assembly. In embodiments, the cleaning member is formed of an absorbent material and the cleaning device includes a fluid delivery tube to supply fluid from the handle component to the absorbent material of the tool assembly to improve cleaning capabilities of the tool assembly. The presently disclosed lens cleaning device facilitates cleaning of the lens of an endoscope within a body cavity at the operative.

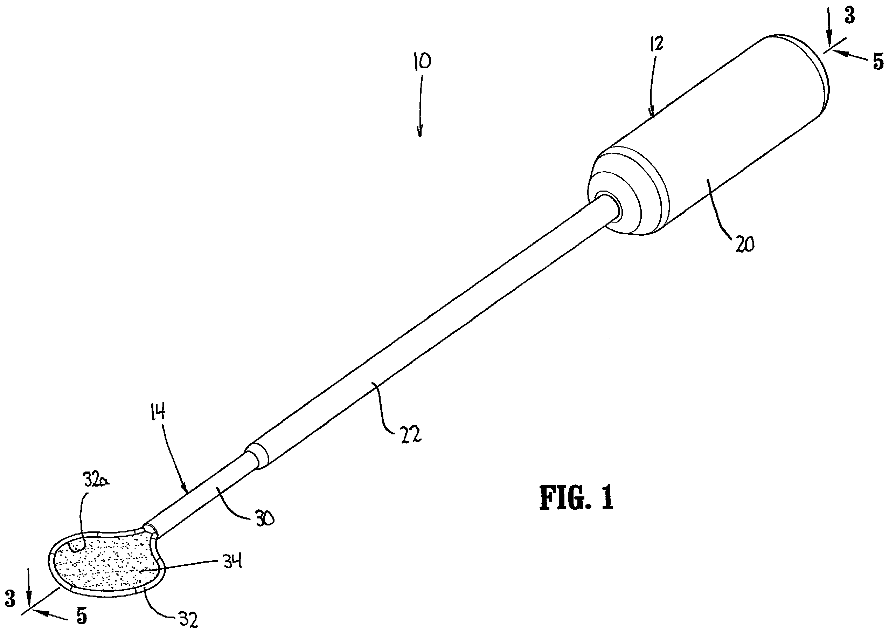

FIGS. 1-6 illustrate an exemplary embodiment of the presently disclosed intraoperative lens cleaning device shown generally as cleaning device 10. Referring to FIGS. 1-5, the cleaning device 10 includes a handle component 12 and a tool assembly 14 that is coupled to a distal portion of the handle component 12. The handle component 12 includes grip portion 20 and an elongate body portion 22. The grip portion 20 and the elongate body portion 22 can be integrally formed from a substantially rigid material, e.g., stainless steel, plastic, etc. Alternately, the grip portion 20 and the elongate body portion 22 can be formed separately and coupled together using any known suitable coupling technique.

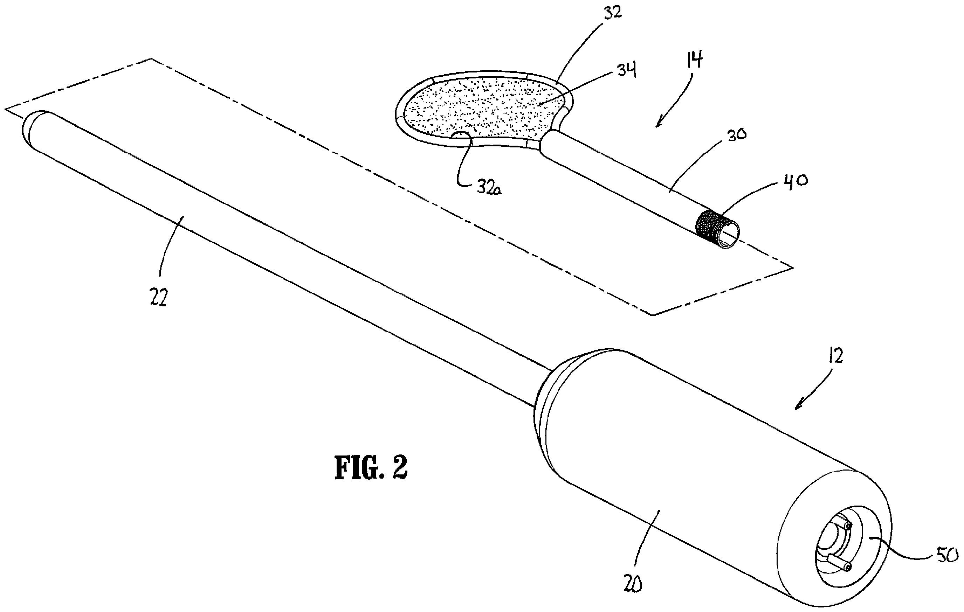

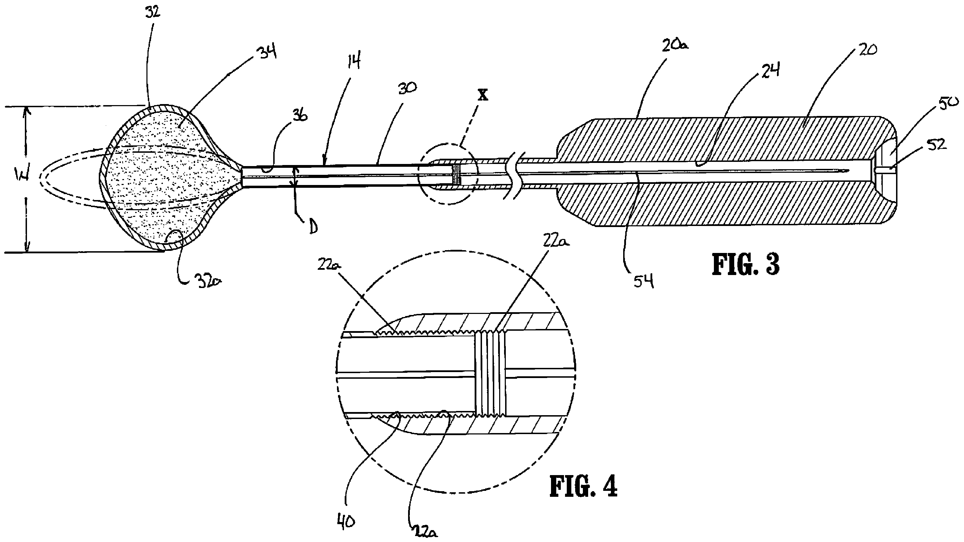

Referring to FIGS. 2-4, the grip portion 20 and the elongate body portion 22 define an internal bore 24 (FIG. 3) that extends through the handle component 12 to a distal portion of the elongate body portion 22. The distal end of the elongate body portion 22 includes an internal thread 22a that is positioned about the internal bore 24. The internal thread 22a is provided to releasably couple the handle component 12 to the tool assembly 14 as described in detail below. In embodiments, the grip portion 20 includes a slip-resistant covering 20a that enables a clinician to more easily grasp the cleaning device 10 in wet conditions. The slip resistant covering 20a can be formed from a plurality of known materials including soft rubbers or the like.

The tool assembly 14 includes a proximal body portion 30, a distal loop 32, and a cleaning member 34 that is supported on the distal loop 32. The proximal body portion 30 is tubular and defines an internal bore 36 that communicates with the internal bore 24 of the handle component 12. An external thread 40 is formed about a proximal end of the proximal body portion 30. The external thread 40 is adapted to engage the internal thread 22a of the elongate body 22 of the handle component 12 to releasably couple the tool assembly 14 to the handle component 12. Alternately, coupling devices other than threads can be provided to releasably couple the tool assembly 14 to the elongate body portion 22 of the handle component 12. For example, the tool assembly 14 can be coupled to the handle component 12 using interlocking structure, a bayonet type coupling, etc.

The distal loop 32 defines an opening 32a and is secured to and projects distally from a distal portion of the proximal body portion 30 of the tool assembly 14. The distal loop 32 is formed from a deformable and resilient material that can be deformed to be received within a small diameter trocar-cannula assembly, e.g., a 5mm or 10mm trocar/cannula assembly. In embodiments, the distal loop, in an undeformed configuration, has a width "W" (FIG. 3) that is substantially larger than the diameter "D" of the proximal body portion 30. For example, the width "W" of the distal loop 32 of the tool assembly 14 can be 1.5 to 10 times greater than the diameter "D" of the proximal body portion 30 of the tool assembly 14. In certain embodiments, the width "W" of the distal loop 32 of the tool assembly 14 can be 4-8 times greater than the diameter "D" of the proximal body portion 30 of the tool assembly 14. Alternately, other dimensions are also envisioned. In embodiments, the distal loop 32 is formed from a metallic material such as spring steel. Alternately, other materials can be used to form the distal loop 32 including metals, polymers, and/or composites that have the requisite characteristics.

The elongate body portion 22 of the handle component 12 and the proximal body portion 30 of the tool assembly 14 are dimensioned to be received within a small diameter trocar-cannula assembly, e.g. 5mm or 10mm trocar-cannula assembly. Although the distal loop portion 32 of the tool assembly 12 is formed of a deformable material and can be deformed to fit in a small diameter trocar-cannula assembly, the proximal body portion 30 of the tool assembly 14 and the elongate body portion 22 of the handle component are formed of a substantially rigid material. Thus, the proximal body portion 30 of the tool assembly 14 and the elongate body portion 22 of the handle component 12 can have a diameter no greater than the size of the trocar-cannula assembly to be used during a surgical procedure.

The cleaning member 34 of the tool assembly 10 of the cleaning device 10 is supported on the distal loop 32 over the opening 32a and is formed of a biocompatible material. In embodiments, the biocompatible material can be absorbent to receive a cleaning solution and can also be substantially transparent so as not to obstruct visualization of the operative site by the clinician. In some embodiments, the biocompatible material is a biocompatible fabric or a microfiber pad. In certain embodiments, the cleaning member 34 is dimensioned to be substantially taut and/or in tension when the distal loop is in its undeformed configuration (FIG. 1) to provide a surface for cleaning a lens of an endoscope as described in further detail below.

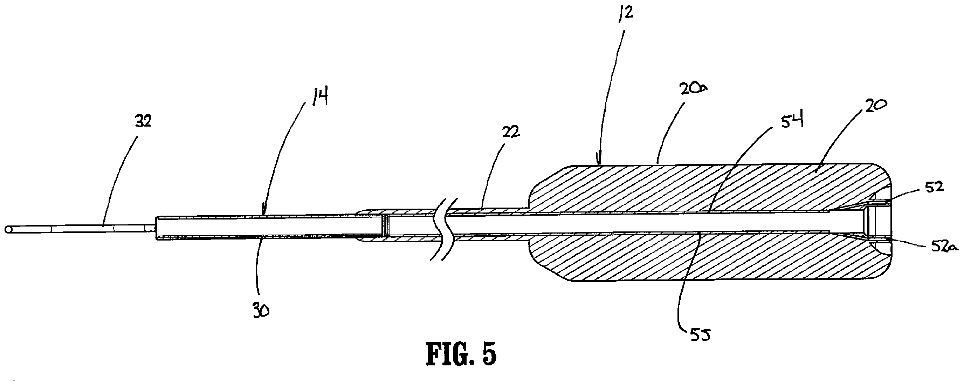

In embodiments, the cleaning device 10 includes a fluid delivery member 54 and a fluid suction tube 55 (FIG. 5). The fluid delivery member 54 may include capillary tubes or a wicking member that is configured to supply a cleaning fluid from the handle component 12 to the cleaning member 34 of the tool assembly 14 to improve cleaning capabilities of the tool assembly 14. Similarly, the fluid suction device 55 may include a tube that extends between the handle component 12 and the cleaning member 34 of the tool assembly 14.

In embodiments, the grip portion 20 of the handle component 12 of the cleaning device 10 includes an opening 50 that communicates with the internal bore 24 of the handle component 12. The grip portion 20 includes a fluid inlet port 52 and a fluid suction port 52a that extend proximally from within the opening 52. The ports 52 and 52a communicate with the fluid delivery member 54 and a fluid suction tube 55, respectively, that are positioned within the internal bores 24 and 32 of the handle component 12 and the tool assembly 14 (FIG. 5). The delivery member 54 and the fluid suction tube 55 each have a distal end that is a positioned adjacent to or in contact with the cleaning member 34 such that a cleaning solution supplied to the inlet port 52 can be delivered to the cleaning member 34 or debris positioned on the cleaning member 34 can be removed from the cleaning member 34.

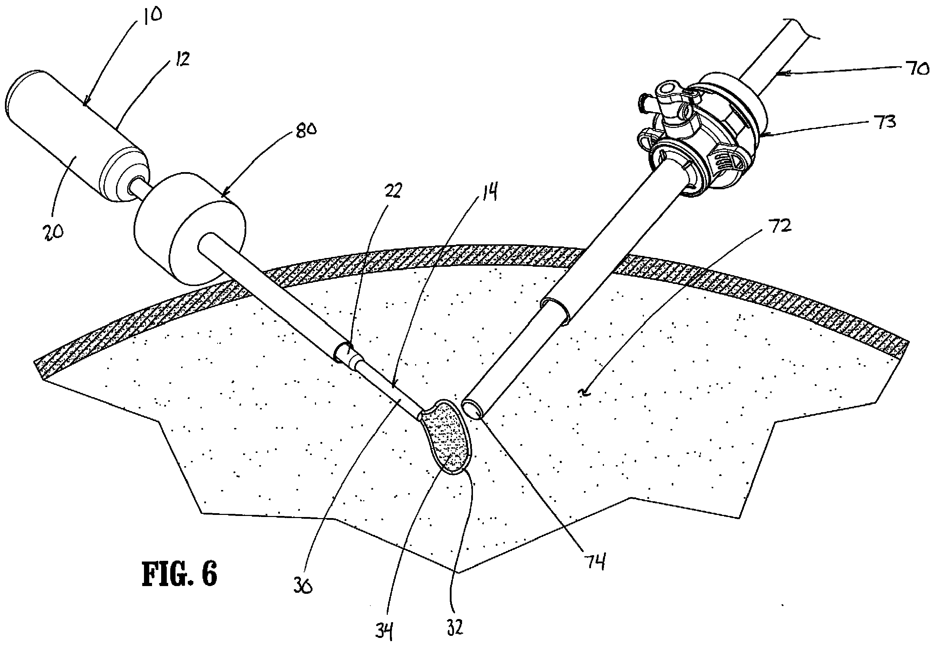

Referring to FIG. 6, during an endoscopic surgical procedure, an endoscope 70 can be introduced into a body cavity 72 of a patient "P" through a first trocar-cannula assembly 73. The endoscope 70 includes a lens 74 that allows a clinician to visualize the operative site during the surgical procedure. In preparation for an endoscopic surgical procedure, the body cavity 72 is insufflated with CO₂ gas to provide space in which the clinician can perform the surgical procedure. During the surgical procedure, tissue is also heated or cauterized using a variety of surgical instrumentation to dissect, ligate, or treat tissue in some manner. As such, the conditions in the body cavity 72 at the operative site are cold, humid, and smoky. This results in deposits on the lens 74 of the endoscope 70 that obstruct the clinician's ability to visualize the operative site.

In order to clean the lens 74 of the endoscope 70, the cleaning device 10 can be inserted through a second trocar cannula assembly 80 to position the cleaning member 34 of the tool assembly 14 adjacent the lens 74 of the endoscope 70. When the lens 74 of the endoscope 70 becomes smudged, splattered with blood, or covered by debris in some way to obstruct the clinician's ability to visualize the operative site, the cleaning device 10 can be manipulated by a clinician to wipe the lens 74 of the endoscope with the cleaning member 34 of the cleaning device 10. More particularly, a clinician can grasp the grip portion 20 of the handle component 12 and move the cleaning device 10 such that the cleaning member 34 is rubbed across the lens 74 of the endoscope 70 to remove obstructions and debris from the lens 74. As described above, to improve the cleaning capabilities of the cleaning member 34, a cleaning solution can be delivered to the cleaning member 34 via the inlet ports 52 and the delivery members 54.

During an endoscopic surgical procedure, the lens 74 of the endoscope 70 may require cleaning a number of times. This may be especially true during surgical procedures that require the use of electro-surgical instruments. The presently disclosed cleaning device 10 can remain within the body cavity 72 during the surgical procedure to clean the lens 74 of the endoscope 70 multiple times. This obviates the need to repeatedly remove the endoscope 70 from the body cavity 72 to clean the endoscope 70. In embodiments, debris on the cleaning member 34 can be removed from the cleaning member 34 through the fluid suction port 52a and the fluid suction tube 55 (FIG. 5). As described, removal of the endoscope from the body cavity for cleaning and subsequently reintroducing the endoscope to the body cavity may result in loss of insufflation gases, an increase in the chance of infection, an increase in the length of the surgical procedure, and repeated loss of visualization of the operative site.

If necessary, the cleaning device 10 can be removed from the body cavity and replaced by uncoupling, e.g., unscrewing, the tool assembly 14 from the handle component 12. In addition, after the surgical procedure is completed, the tool assembly 14 can be removed from the handle component 12 and discarded. The handle component 12 can be sterilized, e.g., autoclaved, and a new sterile tool assembly 14 can be attached to the handle component 14 to facilitate reuse of the cleaning device 10 in another surgical procedure.

Persons skilled in the art will understand that the devices and methods specifically described herein and illustrated in the accompanying drawings are non-limiting exemplary embodiments. It is envisioned that the elements and features illustrated or described in connection with one exemplary embodiment may be combined with the elements and features of another without departing from the scope of the present disclosure. As well, one skilled in the art will appreciate further features and advantages of the disclosure based on the above-described embodiments. Accordingly, the disclosure is not to be limited by what has been particularly shown and described, except as indicated by the appended claims.

The invention may be described by reference to the following numbered paragraphs:-
1. An intraoperative lens cleaning device comprising:
   a handle component including a grip portion and an elongate body portion, the elongate body portion and the grip portion defining an internal bore, the elongate body portion extending distally from the grip portion and including a distal portion having a first coupling member;
   a tool assembly including a proximal body portion that defines an internal bore and a distal loop portion, the internal bore of the proximal body portion communicating with the internal bore of the handle component, the proximal body portion including a second coupling member that is configured to engage the first coupling member to releasably couple the tool assembly to the handle component, the distal loop portion defining an opening and being formed of a resilient material that is deformable to a deformed configuration to be received within a trocar cannula assembly; and
   a cleaning member formed from a biocompatible material, the cleaning member being supported on the distal loop to cover the opening.
2. The intraoperative lens cleaning device of paragraph 1, wherein the cleaning member is formed of an absorbent material.
3. The intraoperative lens cleaning device of paragraph 1, wherein the cleaning member is formed of a biocompatible fabric.
4. The intraoperative lens cleaning device of paragraph 1, wherein the cleaning member is formed of cotton fibers.
5. The intraoperative lens cleaning device of paragraph 1, wherein the cleaning member is formed of a transparent material.
6. The intraoperative lens cleaning device of paragraph 1, wherein the proximal body portion has a diameter "D" and the distal loop portion has a width "W", the width "W" being greater than the diameter "D".
7. The intraoperative lens cleaning device of paragraph 6, wherein "W" is 5 to 10 times greater than "D".
8. The intraoperative lens cleaning device of paragraph 6, wherein "W" is 4 to 8 times greater than "D".
9. The intraoperative lens cleaning device of paragraph 1, wherein the grip portion includes a slip-resistant covering.
10. The intraoperative lens cleaning device of paragraph 9, wherein the slip-resistant covering is formed from rubber.
11. The intraoperative lens cleaning device of paragraph 1, further including a fluid delivery member supported within the internal bore of the handle component and the internal bore of the proximal body portion of the tool assembly, the fluid delivery member including a proximal portion and a distal portion, the distal portion being positioned adjacent to the cleaning member.
12. The intraoperative lens cleaning device of paragraph 11, wherein the fluid delivery device includes a capillary tube.
13. The intraoperative lens cleaning device of paragraph 1, further including a fluid suction port and a fluid suction tube, the fluid suction port communicating with a proximal end of the fluid suction tube, the fluid suction tube having a distal end in fluid communication with the cleaning member of the tool assembly.
14. The intraoperative lens cleaning device of paragraph 11, wherein the handle component includes a fluid inlet port, the fluid inlet port communicating with a proximal portion of the fluid delivery member.
15. The intraoperative lens cleaning device of paragraph 1, wherein the first and second coupling members include first and second screw threads, respectively.
16. A method of performing a surgical procedure comprising:
   inserting an endoscope through a first incision into a body cavity of a patient;
   inserting a cleaning device through a second incision into the body cavity of the patient to position a tool assembly of the cleaning device adjacent a lens of the endoscope;
   manipulating the cleaning device to position a cleaning member of the tool assembly into contact with the lens of the endoscope within the body cavity to clean the lens of the endoscope within the body cavity.
17. The method of paragraph 16, further including supplying a cleaning solution to the cleaning member through a delivery member of the cleaning device.
18. The method of paragraph 16, wherein inserting the cleaning device through the second incision includes inserting the cleaning device through a trocar cannula assembly having a 5mm diameter positioned within the second incision.
19. The method of paragraph 18, wherein inserting the cleaning device through the 5mm trocar includes deforming the cleaning member of the cleaning device from a dimension greater than the diameter of the trocar cannula assembly to a dimension smaller than the diameter of the cannula trocar assembly.

## Claims

1. An intraoperative lens cleaning device comprising:
a handle component including a grip portion and an elongate body portion, the elongate body portion and the grip portion defining an internal bore, the elongate body portion extending distally from the grip portion and including a distal portion having a first coupling member;
a tool assembly including a proximal body portion that defines an internal bore and a distal loop portion, the internal bore of the proximal body portion communicating with the internal bore of the handle component, the proximal body portion including a second coupling member that is configured to engage the first coupling member to releasably couple the tool assembly to the handle component, the distal loop portion defining an opening and being formed of a resilient material that is deformable to a deformed configuration to be received within a trocar cannula assembly; and
a cleaning member formed from a biocompatible material, the cleaning member being supported on the distal loop to cover the opening.

2. The intraoperative lens cleaning device of claim 1, wherein the cleaning member is formed of an absorbent material.

3. The intraoperative lens cleaning device of claim 1 or claim 2, wherein the cleaning member is formed of a biocompatible fabric.

4. The intraoperative lens cleaning device of claim 1 or claim 2, wherein the cleaning member is formed of cotton fibers.

5. The intraoperative lens cleaning device of any of claims 1 to 3, wherein the cleaning member is formed of a transparent material.

6. The intraoperative lens cleaning device of any preceding claim, wherein the proximal body portion has a diameter "D" and the distal loop portion has a width "W", the width "W" being greater than the diameter "D".

7. The intraoperative lens cleaning device of claim 6, wherein "W" is 5 to 10 times greater than "D".

8. The intraoperative lens cleaning device of claim 6, wherein "W" is 4 to 8 times greater than "D".

9. The intraoperative lens cleaning device of any preceding claim, wherein the grip portion includes a slip-resistant covering.

10. The intraoperative lens cleaning device of claim 9, wherein the slip-resistant covering is formed from rubber.

11. The intraoperative lens cleaning device of any preceding claim, further including a fluid delivery member supported within the internal bore of the handle component and the internal bore of the proximal body portion of the tool assembly, the fluid delivery member including a proximal portion and a distal portion, the distal portion being positioned adjacent to the cleaning member.

12. The intraoperative lens cleaning device of claim 11, wherein the fluid delivery device includes a capillary tube.

13. The intraoperative lens cleaning device of any preceding claim, further including a fluid suction port and a fluid suction tube, the fluid suction port communicating with a proximal end of the fluid suction tube, the fluid suction tube having a distal end in fluid communication with the cleaning member of the tool assembly.

14. The intraoperative lens cleaning device of claim 11, wherein the handle component includes a fluid inlet port, the fluid inlet port communicating with a proximal portion of the fluid delivery member.

15. The intraoperative lens cleaning device of any preceding claim, wherein the first and second coupling members include first and second screw threads, respectively.
